# EUROPEAN PATENT APPLICATION

(11) **EP 1 790 350 A1**
(43) Date of publication of application: **30.05.2007**
(21) Application number: 05447260.0
(22) Date of filing: 24.11.2005
(51) Int. Cl.: A61K 35/36, A61L 27/38

(54) **Use of keratinocyte composition for the treatment of restenosis**

(71) Applicant: Interstitial Therapeutics, 1206 Geneva (CH)
(72) Inventor: Popowski, Youri, 1206 Geneva (CH)
(74) Representative: De Clercq, Ann G. Y.

(57) **Abstract**

The present invention relates to a method for treating and/or preventing restenosis in a subject comprising the step of administering locally to a subject a composition comprising keratinocytes. Administration is preferably by way of a catheter disposed with an inflatable balloon, such as a double or dumbell-shaped balloon.

## Description

### BACKGROUND TO THE INVENTION

Restenosis constitutes one of the main limitations in the success of percutaneous interventions, such as, for example, angioplasty for opening up a coronary artery or other vessel which has become blocked due to build up of deposits on the vessel wall. During angioplasty, a balloon catheter is introduced into the region of the blockage, and is hydraulically inflated in order to open the obstructed vessel lumen by compressing the closed wall. Commonly, a stent - an expandable wire mesh - is employed on the balloon and is implanted to support the new stretched open position of the vessel. However, the damage caused to the wall of the vessel during angioplasty causes restenosis *i.e.* proliferation of cells in the vessel wall which over a period of time contributes to reblocking of the vessel.

Where stents are deployed during angioplasty, they may be coated with drugs to prevent restenosis, such drugs being, for example, sirolimus, everolimus or paclitaxel. However, the problems associated with coated stents include the prolonged time over which the drug is released and the protracted irritation of the vessel wall by the polymer which covers the stent surface. Indeed, because of the efficacy of the drug in preventing restenosis, endothelial cell proliferation and adhesion on the stents struts may be inhibited in some patients, leaving the stent struts apparent as a foreign body inside the vessel for months and requiring protracted anti-platelet therapy (for instance with clopidogrel). After stopping the anti-platelet therapy, the risk of acute vessel thrombosis may persist for instance at 18 months or even later. The problem of delayed acute stent thrombosis may be relatively elevated especially when several stents are implanted in one patient (up to 2 % of risk of late acute thrombosis and sudden death).

Some biodegradable stents are being developed, being made from, for example, polymers, magnesium alloys. They will remain in a vessel for several months only, progressively degrade and disappear. In this case, there is an interest in planning the reconstruction of the vessel wall, instead of inhibiting vessel wall healing with drugs such as paclitaxel, sirolimus or everolimus.

There is a need for treating restenosis which avoids the complications of deploying a stent, or coated stent mentioned above, and which provides a once-only treatment for vessel wounds.

### BRIEF DESCRIPTION OF THE FIGURE

**Fig. 1** Depicts view of a double balloon catheter positioned in a vessel of a subject, said balloons flanking the site of a wound created by an intervention.
**Fig. 2:** Depict views of a dumbbell-shaped balloon catheter positioned in a vessel of a subject, where outer members flank the site of a wound created by an intervention.

### DETAILED DESCRIPTION OF THE INVENTION

Unless defined otherwise, all technical and scientific terms used herein have the same meaning as is commonly understood by one of skill in the art. All publications referenced herein are incorporated by reference thereto.

The articles "a" and "an" are used herein to refer to one or to more than one, *i.e.* to at least one of the grammatical object of the article. By way of example, "an integrin activator" means one integrin activator or more than one integrin activator.

The recitation of numerical ranges by endpoints includes all integer numbers and, where appropriate, fractions subsumed within that range (e.g. 1 to 5 can include 1, 2, 3, 4 when referring to, for example, a number of integrin activators, and can also include 1.5, 2, 2.75 and 3.80, when referring to, for example, concentrations).

The present invention relates to a method for the treatment and/or prevention of restenosis in subject a comprising the step of administering locally a composition comprising keratinocytes.

The inventors have found that such composition is effective in healing and sealing the wounds caused by interventions in vessels, and prevents formation of restenosing cell masses. A coating with keratinocytes will play the role of active endothelial coating and will prevent restenosis and occurrence of acute thrombosis. The composition avoids the need for using a coated stent. It can be applied to the region of treatment where a non-coated stent is deployed. It can also be applied to the region of treatment where a biodegradable stent is deployed. It can be deployed in a coronary vessel or in a peripheral vessel. Alternatively, it may be applied to the region of treatment where no stent is present, so avoiding the use of a stent altogether. The keratinocytes integrate into the surface of the wound where they appear to establish and colonise as would endothelial cells. They prevent restenosis, allowing the wound to heal without scarring as it would happen when seeding endothelial cells. Therefore, the treatment is required only once.

The composition is administered locally. It may be administered in the region where restenosis is expected to arise, or in the region where evidence of initial restenosis is apparent (e.g. recurrence after a first unsuccessful angioplasty). It may administered in the region of the wound caused by the intervention. For example, during angioplasty, a coronary vessel may be expanded by an inflatable balloon, resulting in damage to the wall of the vessel. The surgeon would be aware that restenosis would arise in the region of the damage, and would afterwards apply in the region of the wound the composition in accordance with the present invention.

One embodiment of the present invention is a method for treating and/or preventing restenosis in a subject comprising the step of administering locally a composition comprising keratinocytes. The keratinocytes can be freshly harvested and/or cultured as described below.

Another embodiment of the present invention is a method for treating and/or preventing restenosis in a subject comprising the step of administering locally a composition comprising keratinocytes after performing an angioplasty.

Another embodiment of the present invention is a method for treating and/or preventing restenosis in a subject treated by angioplasty, comprising the step of administering locally a composition comprising keratinocytes .

Another embodiment of the present invention is a method for treating and/or preventing restenosis in a subject having undergone a surgical intervention or minimal invasion on a vessel comprising the step of administering locally a composition comprising keratinocytes. A vessel refers to any internal walled duct such as, for example, an artery, vein, ureter, urethra. The present invention finds its most common application in a native artery, vein, or in a grafted vessel.

Another embodiment of the present invention is a method for treating and/or preventing restenosis in a subject having a wound in the internal wall of a vessel, comprising the step of administering to the region of the wound a composition comprising keratinocytes.

Another embodiment of the present invention is a use of a composition comprising keratinocytes for the preparation of a medicament for the treatment and/or prevention of restenosis. Another embodiment of the present invention is a use of a composition comprising keratinocytes for the preparation of a medicament for the treatment and/or prevention of restenosis, where said composition is administered locally. Another embodiment of the present invention is a use of a composition comprising keratinocytes for the preparation of a medicament treating and/or preventing restenosis in a subject after performing an angioplasty, wherein said composition is administered locally. Another embodiment of the present invention is a use of a composition comprising keratinocytes for the preparation of a medicament for treating and/or preventing restenosis in a subject treated by angioplasty, wherein said composition is administered locally. Another embodiment of the present invention is a use of a composition comprising keratinocytes for the preparation of a medicament for treating and/or preventing restenosis in a subject having undergone a surgical intervention or minimal invasion in a vessel; the composition may be administered locally. Another embodiment of the present invention is a use of a composition comprising keratinocytes for treating and/or preventing restenosis in a subject having a wound in the internal wall of a vessel; the composition may be administered to the region of the wound.

### Composition

The composition described herein comprises keratinocytes. Keratinocytes are cells derived from the epidermal cells in the skin. Specifically they originate in the basal layer of the epidermis from the division of keratinocyte stem cells. In the skin, they eventually store keratin, the upper layers of keratinised skin providing a physical and waterproof barrier in the upper most superficial dead cells of the stratum corneum. In the present invention, the keratinocyte cells adhere to the vessel wall, in particular to the surface of the wound where they form a protective barrier over the wound in the same way as endothelial cells, and appear to inhibit restenosis by rapidly covering the wounded area thereby preventing the migration of smooth muscle cells from the media. It is surprising that cells taken from the skin, normally destined for proliferation into epidermal cells form a protective barrier in the non-epidermal tissue of a duct interior and effect repair of the wound without subsequent restenosis.

The keratinocytes may be cultured keratinocytes *i.e.* propagated keratinocytes taken from the subject being treated. The cultured keratinocytes may be prepared according to procedures established for the preparation of keratinocytes for the treatment of burns. For example, a skin biopsy may be processed according to the keratinocyte isolation method of Rheinwald and Green (Rheinwald J.G., Green H.: Cell serial cultivation of strains of human epidermal keratinocytes: the formation of keratinizing colonies from single cells. Cell, 6: 331-44, 1975), which is described in the Examples.

The keratinocytes may also be freshly harvested keratinocytes. These are cells taken from the subject being treated, but are not propagated in culture. The harvested keratinocytes may be prepared according to procedures established for the preparation of keratinocytes for the treatment of burns (*e.g.* Navarro, F.A., Stoner, M.L., Park, C.S. et. al. Sprayed keratinocyte suspensions accelerate epidermal coverage in a porcine microwound model. J. Burn Care & Rehabilitation. Nov.-Dec. 21(6): 513-518, 2000.). For example, a skin biopsy is taken from the subject and treated with trypsin to split epidermis from dermis. The cell suspension is rinsed several times until only residues of trypsine remain in the dish. The isolated cells can then directly be applied to the vessel wall using an appropriate medical device after optionally being stimulated for ECM receptor activation.

A commercial medical device, ReCell^{®}, facilitates harvesting of autologous skin cells. Developed as an 'off the shelf kit, ReCell^{®} enables a thin split thickness biopsy, taken at the time of the procedure, to be processed into an immediate cell population dedicated for delivery onto the skin wound surface. ReCell^{®} is currently used to treat a number of injuries such as small skin burns and scalds, application on donor sites for minimised scarring, reconstruction of skin scars and other skin defects, prophylactic use in cosmetic skin resurfacing procedures such as laser treatments for wrinkles. The present invention envisages the use of such biopsy-processing devices.

Several commercial products comprising non-cultured keratinocytes available for the treatment of burns may be more suitable for use in the present invention, and include, for example, ReCell harvested cells ® (Clinical Cell Culture, UK). The composition may comprise such products.
One therapeutic advantage of using freshly harvested, non-cultured keratinocytes is they may contain adhesion molecules (ECM receptors) on their surface for several hours after being harvested. Such adhesion molecules enhance binding of keratinocytes (and endothelial cells) to the treated vessel wall.

Certain cells, such as for example, endothelial cells and keratinocytes adhere to the extracellular matrix (ECM) through interactions with cell surface heterodimeric integrins (*e.g.* aVb3, b1 integrins). Integrins are implicated in cell adhesion, migration and homeostasis. An important feature is their ability to adopt different affinity states that can be regulated by a variety of intra- and extracellular factors. Several transmembrane truncated integrin subunits may stably associated to form a functional receptor, for instance the soluble recombinant alphavbeta3 in endothelial cells.

The advantage of keratinocytes over circulating pro-genitor endothelial cells is that keratinocytes possess a high density of ECM receptors at their surface when freshly harvested, allowing adherence of keratinocytes to the vessel wall.

Compositions prepared from directly harvested, non-cultured keratinocytes may comprise other epidermal cells types from the epidermal strata. The majority of cells are typically keratinocytes (more than 90%), the remainder being melanocytes, Langerhans' cells, some fibroblasts and endothelial cells.

A subpopulation (up to 25% depending on the biopsy site) of the keratinocytes isolated from the basement membrane express particular ECM receptors that enable them to adhere rapidly to ECM molecules. The inventors have observed that one of the ECM proteins for which both keratinocytes and endothelial cells show a high affinity is laminin-5. Another such ECM protein is fibronectin. The inventors have observed that keratinocytes with highly active adhesion receptors will actively bind to laminin-5 among others when placed in presence of this protein. Therefore, it is an aspect of the invention that the composition comprises keratinocytes provided with ECM receptors able to bind laminin-5 or fibronectin. It is a further aspect of the invention that the composition is enriched in keratinocytes provided with ECM receptors able to bind laminin-5.

It is not at all known to apply keratinocytes on an arterial wall. Moreover, the use of keratinocytes to prevent restenosis was never evocated before. However, due to their affinity to some similar ECM proteins, both endothelial cells and keratinocytes will adhere to the vessel wall.

### Activating ECM receptors

Freshly harvested or cultured keratinocytes may be conditioned by certain factors which increase their adhesiveness to the vascular wall. For instance, hepatocyte growth factor/scatter factor (HGF/SF) selectively promotes cell adhesion on laminins 1 and 5, fibronectin, and vitronectin through a P13-K-dependent mechanism (Trusolino L. HGF/scatter factor selectively promotes cell invasion by increasing integrin avidity. The Faseb Journal, 14: 1629-40, 2000). This phenomenon is mediated by multiple integrins including b1, b3, b4, and b5. HGF/SF triggers clustering of all these integrins at actin-rich adhesive sites and lamellipodia but does not quantitatively modify their membrane expression. These data suggest that HGF/SF promotes cell adhesion by increasing the avidity of integrins for their specific ligands. One aspect of the present invention, therefore, is the pre-treatment of keratinocytes by HGF/SF.

Several other peptides or proteins may stimulate integrin activation and cell adhesion to the ECM in a dose dependent manner, some with an antagonistic phase at high concentrations, and an agonistic phase at low concentrations. Examples of such substances include, cyclic RGD-peptide containing the Arg-Gly-Asp sequence for alphavbeta3 endothelial integrin, soluble integrin ligands *e.g.* fibronectin for keratinocytes. These may be present in the composition of the invention in nanomolar concentrations *e.g.* at a concentration equal to or greater than 1, 10, 50, 100, 200, 400, 600, 800, 900 nM, or a value in the range between any two of the aforementioned values.

The affinity between keratinocytes and the vessel wall can also be modulated by divalent cations. For example, magnesium ions and calcium ions may be used to inhibit the interaction, while manganese ions may increase affinity. The modulation is though to arise at the level of integrin activity.

Manganese ions have the most dramatic effect on integrin affinity, since its binding to cation coordination sites activates integrins to maximal affinity. For instance, in the presence of intermediate levels of Mn²⁺ (0.01-0.05 mM), a monoclonal antibody (mAb 9EG7) stimulates strong dose-dependent cell adhesion for several cell types. The effect of 9EG7 grows as Mn²⁺ is increased from 0.01 to 0.05 mM. However at higher Mn²⁺ levels (0.1 mM), Mn²⁺ itself already has a maximal stimulatory effect, such that the effect of 9EG7 becomes less evident (Bazzoni, G., Shih, D. T., Buck, C. A. and Hemler, M. E. (1995). Monoclonal antibody 9EG7 defines a novel beta 1 integrin epitope induced by soluble ligand and manganese, but inhibited by calcium. J. Biol. Chem. 270, 25570-25577). On the contrary, calcium ions inhibit several integrin functions (*e.g.* beta 1 integrin) because it prevents the appearance of a conformation favorable to ligand binding (Bazzoni et al, see above).

*In vivo,* manganese ions cannot be attained in concentrations above its dissociation constant for integrins as it is the case in experimental settings, and thus manganese was estimated as not having any biological role in integrin affinity modulation *in vivo* (Bazzoni *et al.* see above, D'Souza, S. E., Haas, T. A., Piotrowicz, R. S., Byers-Ward, V., McGrath, D. E., Soule, H. R., Cierniewski, C., Plow, E. F. and Smith, J. W. (1994). Ligand and cation binding are dual functions of a discrete segment of the integrin beta 3 subunit: cation displacement is involved in ligand binding. Cell 79, 659-667; Hu, D. D., Barbas, C. F. and Smith, J. W. (1996). An allosteric Ca2+ binding site on the beta3-integrins that regulates the dissociation rate for RGD ligands. J. Biol. Chem. 271, 21745-21751;
Smith, J. W., Piotrowicz, R. S. and Mathis, D. (1994). A mechanism for divalent cation regulation of beta 3-integrins. J. Biol. Chem. 269, 960-967). This is circumvented in the present invention by the association of the inflatable balloon (*e.g.* using a double balloon or dumbbell (Genie, Acrostak) balloon) which allows the creation of a temporarily isolated space permitting a high concentration of Mn²⁺ ions (for instance 0.1 mM) to be reached and maintained. Combined with keratinocytes in the site of angioplasty, this greatly enhances cell adhesion to the vessel wall. Therefore, one aspect of the present invention is the composition as described herein, further comprising manganese ions. In another aspect of the invention, the concentration of manganese ions is more than or about 0.02, 0.04, 0.06, 0.08, 0.1, 0.12, 0.14, 0.16, 0.18, 2.0, 2.5, 3.0, 3.5, 4.0, 4.50, 5.0 mM, or a value in the range between any two of the aforementioned values. Manganese ions may take the form of soluble salts with any suitable anion such as, for example, chloride, sulphate, carbonate, and phosphate.

Some cells such as activated leucocytes may adhere very rapidly to ECM, for example, within 2-3 minutes, while others, such as keratinocytes or circulating endothelial cells necessitate longer incubations (hours). Therefore, it is better to pretreat keratinocytes with Mn²⁺ or with soluble integrin ligands before placing them in contact with the vessel wall ECM. Authors have observed that pretreating keratinocytes with a solution of 0.1 mM of Mn²⁺ for 15 minutes sharply improves the apposition of keratinocytes on the vessel wall. The authors have observed that an extensive vessel wall coated with keratinocytes sharply reduces the rate of restenosis in coronary vessels after angioplasty and bare metal stenting. The inventors believe that the risk of acute thrombosis will be sharply reduced after using patients own cells to cover dilated vessels wall. Of course, patients will be accurately selected for this therapy, and those presenting with skin healing diseases (predisposition for cheloid or hypertrophic scar formation) should be avoided.

Once the vessel wall is coated by keratinocytes, smooth muscle cells stop migrating from the media to obliterate the vessel lumen and restenosis is prevented. Moreover, as the vessel and the stent struts will be partially coated by the keratinocytes, the risk of acute thrombosis will be reduced with this new therapy. Finally, when using biodegradable stents made from polymers or magnesium alloys, the risk of delayed acute thrombosis (>1 year later) will be further reduced.

There is another keratinocyte subpopulation, the differentiating strata, which does not bare the ECM receptors. Some of them will be activated by the cations or ligand suspension, others will adhere only by ion-dependent and/or cell-to-cell interactions. Thus, the majority of keratinocytes will adhere to the vessel tissue/wound because of the wide variety of specific (receptor-ligand) and non-specific (ion dependent) interactions.

When using keratinocytes prepared from patient's own skin samples using ReCell, the trypsinisation procedure takes 15-30 minutes to split epidermis from dermis. The isolated cells can then directly be applied to the vessel wall using an appropriate medical device after being stimulated for ECM receptor activation. It is worth mentioning that the standard procedure does not normally take longer than one hour. Such a duration of preparation and treatment complies with the requirements of a catheterization laboratory, for the treatment of coronary or peripheral arteries.

The composition may comprise additional active agents which are known to expedite healing in skin burn patients, such as, for example, antibiotics or antiseptic substances.

The composition may comprise additional active agents which are known to increase the adhesiveness of keratinocytes, such as, for example RGD-peptide, soluble integrin ligands e.g. fibronectin, Mn²⁺ (0.5 to 0.05 mM), etc,

The invention provides for compositions comprising keratinocytes according to the invention admixed with a physiologically compatible carrier. This may be a physiologically acceptable diluent such as water, phosphate buffered saline, or saline. The therapeutic solution may contain a negligible amount of trypsine. The carrier should also maintain the keratinocytes in an active state. The composition may be pharmaceutically acceptable.

The composition may also by mixed with a suitable excipient such as, for example, carbohydrate or protein fillers such as sugars, including lactose, sucrose, mannitol, or sorbitol; starch from corn, wheat, rice, potato, or other plants; cellulose such as methyl cellulose, hydroxypropylmethyl-cellulose, or sodium carboxymethyl cellulose; and gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate. The active compounds may be dissolved or suspended in suitable liquids, such as fatty oils, liquid paraffin, or liquid polyethylene glycol with or without stabilisers.

The composition of the invention may be formulated in aqueous solutions, preferably in physiologically compatible buffers such as Hank's solution, Ringer's solution, or physiologically buffered saline. Substances which increase the viscosity of the composition, such as sodium carboxymethyl cellulose, sorbitol, or dextran can be used. Additionally, the composition may comprise vehicles including fatty oils such as sesame oil, or synthetic fatty acid esters, such as ethyl oleate or triglycerides, or liposomes. Optionally, the composition may also contain suitable stabilisers or agents which increase the solubility of components in the composition to allow for the preparation of highly concentrated solutions.

The compositions of the present invention may be manufactured in a manner known in the art, e.g. by means of conventional mixing, dissolving, granulating, levitating, emulsifying, encapsulating, entrapping processes.

After compositions comprising keratinocytes formulated in an acceptable carrier have been prepared, they may be placed in an appropriate container and labelled for treatment of an indicated condition with information including amount, frequency and method of administration.

### Method of administration

The composition is applied locally to the walls of the vessel in the region to be treated. This can be achieved by known methods. For example, a catheter with a balloon having a microporous inflatable membrane, which balloon when inflated under hydraulic pressure releases composition to the vessel wall. Such balloons are known in the art, for example described in US 6,425,877, which is incorporated herein by reference. Where such a balloon is used, it may also perform the step of opening a constriction in a vessel, such as in an angioplasty procedure. Thus, another embodiment of the present invention is a method of performing an angioplasty comprising the step of opening a constricted vessel wall using a catheter disposed with an inflatable balloon, where said balloon has a microporous outer membrane, which balloon when inflated under hydraulic pressure both opens the constricted vessel and releases composition through said membrane to the vessel wall.

In a preferred embodiment, the composition is delivered in a space created by two inflated balloons which flank the region to be treated, or by a single balloon having a "dumbbell" or "dog-bone" shape, which outer members flank the region to be treated. The balloons or outer members inflate, and contact the wall of the vessel. When sufficiently inflated, they create a sealed chamber into which composition may be provided, without leakage of the composition into the remainder of the vessel.

In one embodiment, a catheter equipped with two balloons may be inserted into the vessel of a subject. The balloons maybe separated on the catheter by a narrow, non-inflatable spacing member. The composition can be injected into the space between the balloons, through the spacing member or from the inside face of either or both balloons.

In another embodiment, a catheter equipped with the dumbbell balloon is inserted into the vessel of a subject. The larger outer members of the balloon may be separated on the catheter by a more narrow inflatable spacing member. The composition can be injected into the space between the outer members, through the spacing member or from the inside face of either or both outer members . Such devices are known in the art, for example, the Genie^{™} manufactured by Acrostak (www.acrostak.com). Other balloon types may be used as well.

When using the Genie balloon, it may be inflated with a solution containing the keratinocytes which emerge from holes located in the distal outer member, fill the space between the proximal and distal balloon parts, and remain in contact with the vessel wall for a determined period. The vascular flow will be interrupted during a period of up to five minutes, until a significant amount of keratinocytes adhere to the vessel wall. In case five minutes is insufficient, time, the composition can be drained, the balloon deflated and the procedure repeated. At the end of treatment, the dumbbell balloon is then deflated and removed. The keratinocytes will easily resist to the blood flow after balloon deflation, will divide and progressively coat all the vessel wall surface, in the same way as endothelial cells recover the surface when an artery heals after angioplasty.

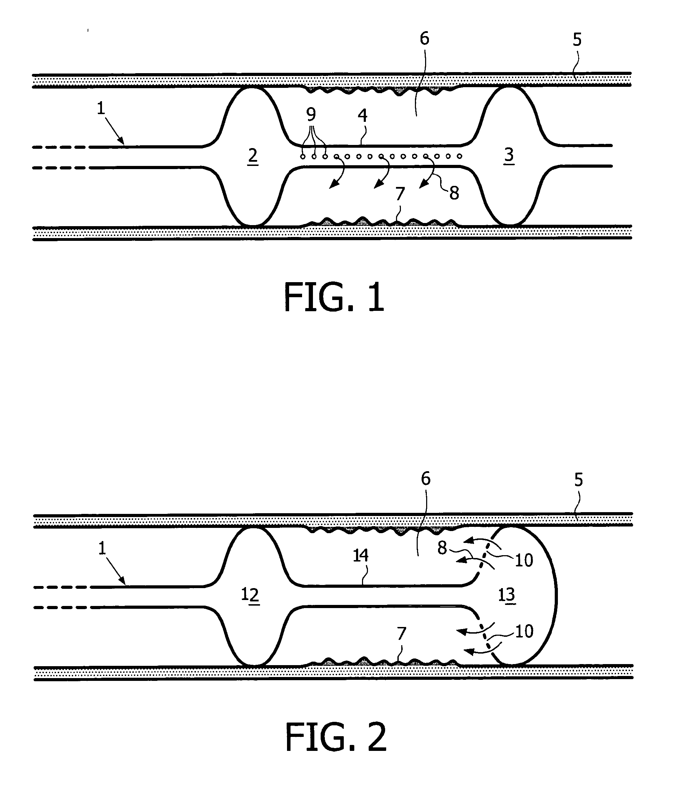

Examples of a double balloon and dumbbell-shaped balloon device are shown in Figures 1 and 2. With reference to **Figure 1,** a catheter **1** is provided with a first **2** and second **3** balloon separated by a non-inflatable spacing member **4.** In the inflated state, the balloons contact the wall of the vessel **5,** creating a space **6** in the vessel **5** between the two balloons **2, 3** which space is sealed-off in an essentially watertight manner from the remainder of the vessel **5.** The wound **7** is located in the space **6** between the balloons. The spacing member **4** is manufactured with a plurality of ports **9** through which composition can be applied **8** in the space **6** between the balloons. During treatment, the balloons **2, 3** are inflated so as to flank the wound **7,** composition is introduced into the space **6** therebetween and allowed to contact the wound **7** for a period of time such as 20 seconds to 5 minutes. Such an exposure may be repeated several times. After exposure, the composition may be withdrawn through the ports **9** and the balloons deflated and the catheter **1** withdrawn. Treatment is thus confined to the region of the wound.

With reference to **Figure 2,** a catheter **1** is provided with a dumbbell balloon, comprising elements of proximal **12** and distal **13** outer members and a spacing member **14.** The elements are arranged to form an inflatable balloon, which inflates in the shape of a dumbbell. In the inflated state, the outer members **12, 13** contact the wall of the vessel **5,** creating a space **6** in the vessel **5** between the two members **12, 13** which space is sealed-off in an essentially watertight manner from the remainder of the vessel **5.** The wound **7** is located in the space **6** between the members **12, 13.** The distal member **13** is manufactured with a plurality of ports **10** through which composition can be applied **8** in the space **6** between the members. During treatment, the members **12, 13** are inflated so as to flank the wound **7,** composition is introduced into the space 6 therebetween and allowed to contact the wound **7** for a period of time such as 20 seconds to 5 minutes. Such an exposure may be repeated several times. After exposure, the composition may be withdrawn through the ports **10** and the balloon deflated and the catheter **1** withdrawn. Treatment is thus confined to the region of the wound.

### Dose

The volume and concentration of composition administered to a subject will depend on the length of the dilated area, the diameter of the vessel, and on the intended exposure time to the composition. Where a double balloon assembly is employed for administration, the volume of composition is generally sufficient to fill the space between the balloon, and can be readily determined by the skilled person. The composition may be diluted from a concentrated stock to form a composition of suitable volume and concentration of keratinocyte.

Generally the composition is administered at a dose between 100 to 1 x 10⁷ keratinocytes per ml of composition for application to the vessel wall. Typical exposure time can vary depending on the size of the vessel surface and on the concentration of keratinocytes. For example, for a vessel surface area to be treated of 225mm², and a keratinocyte concentration of 5000 to 5 000 000 cell/ml, the exposure time might be in the range of 1 to 5 minutes. In a coronary artery blood flow interruption can hardly be superior to 5 minutes. A therapy using activated keratinocytes could be repeated in some selected patients able to withstand long blood flow interruptions. Factors which govern the concentration of keratinocyes include the volume of resected skin, the availability of cultured cells and expense. For example, if the cells are simply harvested (ReCell) or if the time available to prepare cultured cells is less than 3 days, fewer cells would be available, necessitating a more dilute composition and possibly longer exposure time. Factors which govern exposure time and concentration can be readily determined by the skilled person. Factors which modify keratinocyte cell adhesion (soluble integrin ligands, Mn²⁺, ...) may play a major role in determining the exposure time to the keratinocyte solution.

### Medical conditions

The present composition may be used to treat vessels where restenosis is expected to arise, in order to prevent restenosis. It may also be applied to vessels exhibiting the initial stages of restenosis in order to halt the condition after a repeated dilatation. It can be applied in situations where a medical intervention has created a wound on the interior surface of the vessel, such as created, for example, during angioplasty. It can be used where a coated or non-coated stent has already been deployed to ensure proper healing of the wound, so ensuring against restenosis. It may also be used to prevent restenosis as well as acute thrombosis after the interruption of anti-platelet therapy.

### EXAMPLES

The invention is exemplified by the following non-limiting examples.

### Example 1: Preparing cultured Keratinocytes in vitro

Skin biopsies were harvested from burn patients, cleaned with 70% ethanol, and processed according to Rheinwald and Green's keratinocyte isolation method (Rheinwald J.G., Green H.: Cell serial cultivation of strains of human epidermal keratinocytes: the formation of keratinizing colonies from single cells. Cell, 6: 331-44, 1975). Skin biopsies were trypsinized overnight at 4 deg C in a 0.05% trypsin/0.02% EDTA solution. The cells were isolated from the epidermal/dermal junction and plated onto a feeder monolayer of lethally irradiated 3T3 cells. The medium was made up of three parts Dulbecco's modified with Eagle's medium plus one part Ham's F12 medium. The following were added to the mixture: cholera toxin (0.1 nM), hydrocortisone (0.4 pg/ml), triiodo-I-thyronine (20 pM), insulin (5 pg/ml), transferrin (5 pg/ml), fungizone (250 pg/ml), penicillin (1000 IU/ml), streptomycin (1000 p g/ml), and 10% foetal calf serum. Epidermal growth factor (10 ng/ml) was added with the first medium change. Cells were cultured at 37 deg C in a 5% CO atmosphere. The medium was changed every two days. Subconfluent primary cultures were trypsinized and cells were plated in a secondary culture. The confluent secondary cultures were detached from the surface vessel with the neutral protease Dispasell, washed in serum-free medium, and placed on sterile Veloderm®. 15 minutes prior to the treatment, MnCl₂ at a concentration of 0.1 mM was added to the keratinocyte medium in order to optimize keratinocyte adhesion to the vessel wall.

### Example 2: Treatment of a wound

A 51 year old patient presents a 8 mm long stenotic lesion in the proximal part of the right coronary artery. A 3.5 mm diameter balloon is used to dilate the artery and a 16 mm long magnesium alloy stent is inserted and positioned at the level of the former vessel narrowing. A deflated Genie dumbbell balloon is introduced and accurately positioned at the level of the magnesium alloy stent. Ten ml of a solution containing 100 000 keratinocytes/ml with 0.1 mM of Mn²⁺ is used to inflate the Genie balloon. The inflation of the Genie balloon is followed by the flow of the composition through small holes located on the inner wall of the distal balloon enlargement. This contributes to the filling of the space located between both larger balloon parts and the tight contact of the solution of keratinocytes with the vessel wall. A pressure of 2 Atm is necessary to keep the Genie balloon expanded and to let the keratinocyte solution stay in contact with the vessel wall. After 3 minutes of blood flow interruption the patient complains of chest pain. Five mg of morphine is administred sub-cutaneously and the pain resumes. The Genie balloon is deflated and the composition is aspirated with a syringe. Approximately 70 % of the infused composition is extracted back into the syringe. After 6 months the patient is feeling well, and a spiral CT control does not show vessel re-narrowing at the level of the former lesion.

### Example 3: Treatment of a wound

A 76 y old patient presents with a narrowing of his popliteal artery with some pain and claudication after a walk of 300 m. An angiographic examination shows a narrowing of the artery on a length of 5 cm. A percutaneous transcatheter angioplasty (PTA) is performed at the level of the vessel narrowing and a wallstent is inserted at the level of the lesion. A 8 cm long Genie balloon is introduced inside the artery and is accurately placed at the level of the dilated lesion. Thirty ml of a keratinocyte suspension stimulated by nanomolar suspension of fibronectin and by 0.1 mM of Mn²⁺ is infused in the Genie balloon and in the intra-vascular space. The patient does not show any sign of recurrence 8 months later. A Doppler evaluation shows a regular, non interrupted blood flow in the vessel.

### SUMMARY OF SOME EMBODIMENTS OF THE INVENTION

One embodiment of the present invention is a use of a composition comprising keratinocytes for the preparation of a medicament for the treatment and/or prevention of restenosis in a subject, wherein said composition is administered locally.

Another embodiment of the present invention is a use as described above, wherein said keratinocytes are keratinocytes harvested from a skin biopsy of the subject. The keratinocytes are preferably freshly harvested *e.g.* used within 1, 2, or 3 hours of taking the biopsy.

Another embodiment of the present invention is a use as described above, comprising the use of a ReCell® device to process a skin biopsy.

Another embodiment of the present invention is a use as described above, wherein said composition further comprises endothelial cells.

Another embodiment of the present invention is a use as described above, wherein said composition is enriched in keratinocytes provided with ECM receptor able to bind to Laminin-5.

Another embodiment of the present invention is a use as described above, wherein said keratinocytes are keratinocytes cultured from a skin biopsy of the subject.

Another embodiment of the present invention is a use as described above, wherein said keratinocytes are activated using an integrin activator.

Another embodiment of the present invention is a use as described above, wherein said integrin activator comprises manganese ion at a concentration between 0.05 and 0.5 mM and/or soluble integrin ligand at a concentration between 1 and 900 nM. Thus the keratinocytes can be either freshly harvested keratinocytes, or cultured keratinocytes. Both types may be activated using for instance an integrin activator (*e.g.* Mn²⁺).

Another embodiment of the present invention is a use as described above, wherein said composition is admixed with a physiologically compatible carrier and/or suitable excipient.

Another embodiment of the present invention is a use as described above, wherein the concentration of keratinocytes in the composition is between 100 to 10⁷ cells per ml of composition.

Another embodiment of the present invention is a use as described above, wherein said composition is delivered to a vessel by means of a double balloon catheter, where a space between the inflated balloons is sealed from the remainder of the vessel, which space is provided with composition.

Another embodiment of the present invention is a use as described above, wherein said composition is delivered to a vessel by means of a dumbbell-shaped balloon catheter, which balloon comprises a central inflatable spacing region flanked by two outer inflatable members, where a space between said outer inflatable members parts is sealed from the remainder of the vessel, which space is provided with composition.

Another embodiment of the present invention is a use as described above, wherein said composition is delivered to a vessel by means of a catheter disposed with an inflatable balloon having an outer microporous inflatable membrane, which balloon when inflated under hydraulic pressure releases composition through said membrane to the vessel wall.

Another embodiment of the present invention is a kit comprising a composition as defined above.

Another embodiment of the present invention is a kit as described above, wherein said composition is in a concentrated form.

Another embodiment of the present invention is a kit as described above, further comprising a double balloon or dumbbell-shaped balloon catheter.

Another embodiment of the present invention is a method for treating and/or preventing restenosis in a subject comprising the step of administering locally to a subject a composition comprising keratinocytes.

Another embodiment of the present invention is a method as described above, wherein said keratinocytes are freshy harvested keratinocytes from a skin biopsy of the subject.

Another embodiment of the present invention is a method as described above, wherein said keratinocytes are keratinocytes that have been harvested less than 3 hours (or less than 2 hours or less than 1 hour) before on the skin of the subject.

Another embodiment of the present invention is a method as described above, comprising the use of ReCell® to process a skin biopsy.

Another embodiment of the present invention is a method as described above, wherein said composition further comprises endothelial cells.

Another embodiment of the present invention is a method as described above, wherein said composition is enriched in keratinocytes provided with ECM receptor able to bind to Laminin-5.

Another embodiment of the present invention is a method as described above, wherein said keratinocytes are keratinocytes cultured from a skin biopsy of the subject.

Another embodiment of the present invention is a method as described above, wherein said keratinocytes are activated using an integrin activator.

Another embodiment of the present invention is a method as described above, wherein said integrin activator comprises manganese ion at a concentration between 0.05 and 0.5 mM and/or soluble integrin ligand at a concentration between 1 and 900 nM. Thus the keratinocytes can be either freshly harvested keratinocytes, or cultured keratinocytes. Both types may be activated using for instance an integrin activator (*e.g.* Mn²⁺ or soluble integrin ligand).

Another embodiment of the present invention is a method as described above, wherein said composition is admixed with a physiologically compatible carrier and/or suitable excipient.

Another embodiment of the present invention is a method as described above, wherein the concentration of keratinocytes in the composition is between 100 to 10⁷ cells per ml of composition.

Another embodiment of the present invention is a method as described above, wherein said composition is delivered to a vessel by means of a double balloon catheter, where a space between the inflated balloons or the larger balloon parts is sealed from the remainder of the vessel, which space is provided with composition.

Another embodiment of the present invention is a method as described above, wherein said composition is delivered to a vessel by means of a dumbbell-shaped balloon catheter, which balloon comprises a central inflatable spacing region flanked by two outer inflatable members, where a space between said outer inflatable members parts is sealed from the remainder of the vessel, which space is provided with composition.

Another embodiment of the present invention is a method as described above, wherein said composition is delivered to a vessel by means of a catheter disposed with an inflatable balloon having a microporous inflatable membrane, which balloon when inflated under hydraulic pressure releases composition through said membrane to the vessel wall.

Another embodiment of the present invention is a method of performing an angioplasty comprising the step of opening a constricted vessel wall using a catheter disposed with an inflatable balloon, wherein said balloon has an outer microporous inflatable membrane, which balloon when inflated under hydraulic pressure both opens the constricted vessel and releases a composition as defined above through said membrane to the vessel wall.

## Claims

1. Use of a composition comprising keratinocytes for the preparation of a medicament for the treatment and/or prevention of restenosis in a subject, wherein said composition is administered locally.

2. Use according to claim 1, wherein said keratinocytes are keratinocytes harvested from a skin biopsy of the subject.

3. Use according to claim 2, comprising the use of a ReCell® device to process a skin biopsy.

4. Use according to claim 2 or 3, wherein said composition further comprises endothelial cells.

5. Use according to any of claim 2 to 4, wherein said composition is enriched in keratinocytes provided with ECM receptor able to bind to Laminin-5.

6. Use according to claim 1, wherein said keratinocytes are keratinocytes cultured from a skin biopsy of the subject.

7. Use according to any of claims 1 to 6, wherein said keratinocytes are activated using an integrin activator.

8. Use according to claim 7, wherein said integrin activator comprises manganese ion at a concentration between 0.05 and 0.5 mM and/or soluble integrin ligand at a concentration between 1 and 900 nM.

9. Use according to claim 1 or 8, wherein said composition is admixed with a physiologically compatible carrier and/or suitable excipient.

10. Use according to any of claims 1 to 9, wherein the concentration of keratinocytes in the composition is between 100 to 10⁷ cells per ml of composition.

11. Use according to any of claims 1 to 10, wherein said composition is delivered to a vessel by means of a double balloon catheter, where a space between the inflated balloons is sealed from the remainder of the vessel, which space is provided with composition.

12. Use according to any of claims 1 to 10, wherein said composition is delivered to a vessel by means of a dumbbell-shaped balloon catheter, which balloon comprises a central inflatable spacing region flanked by two outer inflatable members, where a space between said outer inflatable members parts is sealed from the remainder of the vessel, which space is provided with composition.

13. Use according to any of claims 1 to 10, wherein said composition is delivered to a vessel by means of a catheter disposed with an inflatable balloon having an outer microporous inflatable membrane, which balloon when inflated under hydraulic pressure releases composition through said membrane to the vessel wall.

14. Kit comprising a composition as defined in any of claim 1 to 10.

15. Kit according to claim 14, wherein said composition is in a concentrated form.

16. Kit according to claim 14 or 15, further comprising a double balloon or dumbbell-shaped balloon catheter.

17. Method for treating and/or preventing restenosis in a subject comprising the step of administering locally to a subject a composition comprising keratinocytes.

18. Method according to claim 17, wherein said keratinocytes are keratinocytes that have been harvested less than 3 hours before on the skin of the subject.

19. Method according to claim 18, comprising the use of ReCell® to process a skin biopsy.

20. Method according to claim 18 or 19, wherein said composition further comprises endothelial cells.

21. Method according to any of claim 18 to 20, wherein said composition is enriched in keratinocytes provided with ECM receptor able to bind to Laminin-5.

22. Method according to claim 17, wherein said keratinocytes are keratinocytes cultured from a skin biopsy of the subject.

23. Method according to any of claims 17 to 22, wherein said keratinocytes are activated using an integrin activator.

24. Method according to claim 23, wherein said integrin activator comprises manganese ion at a concentration between 0.05 and 0.5 mM and/or soluble integrin ligand at a concentration between 1 and 900 nM.

25. Method according to any of claims 17 to 24, wherein said composition is admixed with a physiologically compatible carrier and/or suitable excipient.

26. Method according to any of claims 17 to 25, wherein the concentration of keratinocytes in the composition is between 100 to 10⁷ cells per ml of composition.

27. Method according to any of claims 17 to 26, wherein said composition is delivered to a vessel by means of a double balloon catheter, where a space between the inflated balloons or the larger balloon parts is sealed from the remainder of the vessel, which space is provided with composition.

28. Method according to any of claims 17 to 27, wherein said composition is delivered to a vessel by means of a dumbbell-shaped balloon catheter, which balloon comprises a central inflatable spacing region flanked by two outer inflatable members, where a space between said outer inflatable members parts is sealed from the remainder of the vessel, which space is provided with composition.

29. Method according to any of claims 17 to 27, wherein said composition is delivered to a vessel by means of a catheter disposed with an inflatable balloon having a microporous inflatable membrane, which balloon when inflated under hydraulic pressure releases composition through said membrane to the vessel wall.

30. Method of performing an angioplasty comprising the step of opening a constricted vessel wall using a catheter disposed with an inflatable balloon, wherein said balloon has an outer microporous inflatable membrane, which balloon when inflated under hydraulic pressure both opens the constricted vessel and releases a composition as defined in any of claims 1 to 10 through said membrane to the vessel wall.
